# EUROPEAN PATENT APPLICATION

(11) **EP 4 737 572 A1**
(43) Date of publication of application: **06.05.2026**
(21) Application number: 23942827.9
(22) Date of filing: 29.06.2023
(51) Int. Cl.: C12N 15/62, C07K 7/23, C07K 19/00, C12N 15/70, A61K 39/12, A61K 39/39, C12R 1/19

(54) **PREPARATION METHOD FOR CASTRATING AP205 VIRUS-LIKE PARTICLE SUBUNIT VACCINE**

(71) Applicant: Shenzhen Herz Life Science Technology Co., Ltd, Shenzhen, Guangdong 518107 (CN)
(72) Inventor: ZHA, Lisha, Shenzhen, Guangdong 518107 (CN); ZHOU, Yuhang, Shenzhen, Guangdong 518107 (CN); HUANG, Qian, Shenzhen, Guangdong 518107 (CN); WU, Maobai, Shenzhen, Guangdong 518107 (CN); ZHENG, Qi, Shenzhen, Guangdong 518107 (CN)
(74) Representative: Cabinet Becker et Associés
(86) International application number: PCT/CN2023/103567
(87) International publication number: WO 2025/000310

(57) **Abstract**

The present invention relates to the fields of molecular biology, virology, immunology and medicine, and in particular to a preparation method for a castrating AP205 virus-like particle subunit vaccine.

## Description

### FIELD

The present disclosure relates to the field of biology, virology, immunology and medicine, and particularly relates to a method for preparing an AP205 virus-like particle subunit vaccine for castration.

### BACKGROUND

Castration refers to the removal of the reproductive system of an animal or the loss of sexual function by external means, including the removal of the testes from male individuals and the removal of the ovaries from female individuals and the two together are referred to as gonadectomy. In addition to surgically removing the gonads, castration can also be achieved by local radiation exposure or chemical treatment. After castration is done in a vertebrate, the source of sex hormone secretion is lost, and as a result, the accessory organs of the genital organs, as well as the secondary and tertiary sexual characteristics, may degenerate.

Gonadotropin-releasing hormone (GnRH) is an endogenous polypeptide hormone of animals. A physiological dose of GnRH-I can cause an increase in the concentration of gonadotropins (such as a mild increase in FSH and a significant increase in LH), thereby promoting the synthesis and secretion of sex hormones (such as estradiol, progesterone, and testosterone), facilitating follicular development and maturation leading to ovulation, or promoting testicular development and sperm maturation, and inducing and maintaining secondary sexual characteristics. In addition, GnRH-I can also directly affect the gonads, regulating the synthesis and secretion of gonadal steroid hormones and promoting gametogenesis.

Autologous antigenic proteins are generally difficult to induce antibody responses against self-antigens. A method to improve the vaccination efficiency is to increase the repetitiveness of the applied antigen. Unlike isolated proteins, viruses can induce a rapid and effective immune response both with and without the help of T cells, even in the absence of any adjuvant. Compared with a few isolated proteins, viruses can trigger a much stronger immune response than their separated components. For B-cell responses, it is well known that a key factor in viral immunogenicity is the repetitiveness and order of surface epitopes. Many viruses exhibit quasicrystal surfaces with regularly arranged epitopes that can effectively cross-link epitope-specific immunoglobulin on B cells. Such cross-linking of surface immunoglobulin on B cells serves as a strong activation signal, directly inducing cell cycle progression and the production of IgM antibodies. Moreover, these activated B cells can stimulate T helper cells, which then induce the class switching of IgM antibodies to IgG antibodies in B cells, as well as the generation of long-term B-cell memory, which is the purpose of any vaccination. The structure of viruses is even related to the production of anti-antibodies in autoimmune diseases and constitutes part of the natural response to pathogens. Therefore, antigens presented on the highly organized viral surface are capable of inducing strong antibody responses against the antigen.

Virus-like particles are the major capsid proteins of RNA bacteriophages, which self-assemble from multiple monomers into a novel class of virus-like particles (VLPs) with high immunogenicity. These VLPs do not contain the RNA genome of the bacteriophage and are therefore incapable of replication. However, studies have shown that various polypeptides can be fused to the N-terminus or C-terminus of the virus-like particle protein, and the resulting fusion proteins can form virus-like particles when expressed in a host, typically and preferably in *Escherichia coli.* In addition, it has been found that if the polypeptide contains at least one antigen and the antigen or at least one antigenic epitope thereof is displayed on the outer surface of the assembled VLPs, the formation of VLPs can effectively enhance the immunogenicity of the target antigen. In addition to the reported virus-like particles AP205 and Qβ, there are also various other VLP platforms such as CuMV and tobacco mosaic virus VLPs, all of which have potential for development into castration vaccines.

The *Escherichia coli* expression system is favorable for the efficient expression of virus-like particles. However, to ensure correct protein folding and achieve faster and more efficient expression, the codon sequence of the exogenous nucleic acid needs to be adaptively modified.

### SUMMARY

In view of the problems of the prior art, the present disclosure provides a method for preparing an AP205_{OPT} virus-like particle subunit vaccine for castration. The vaccine can be easily obtained through bacterial culture with high expression yield for convenient industrial production, and the vaccine exhibits strong immunogenicity and induces rapid immune response.

The present disclosure provides a nucleic acid encoding a GnRH-I-AP205 fusion protein, comprising:
a nucleotide sequence of SEQ ID NO: 1; or
a sequence obtained by substitution, deletion, insertion, and/or replacement of one or more nucleotides of SEQ ID NO: 1; or
a sequence having more than 90% amino acid identity to SEQ ID NO: 1.

The nucleic acid provided in the present disclosure may be DNA, RNA, cDNA, or PNA. The DNA form includes cDNA, genomic DNA, or artificially synthesized DNA. The DNA may be single-stranded or double-stranded. The nucleic acid may comprise nucleotide sequences having different functions, such as coding regions and non-coding regions, for example, regulatory sequences (such as promoter or transcription terminator). Topologically, the nucleic acid may be linear or circular. The nucleic acid may, for example, be part of a vector (such as an expression or cloning vector) or a fragment thereof. The nucleic acid may be directly obtained from a natural source or prepared with the aid of recombinant, enzymatic, or chemical techniques. The RNA form refers to, for example, mRNA obtained by transcription of a gene. The gene sequence may adopt a wild-type sequence or be codon-optimized, without limitation in the present disclosure. In the present disclosure, the nucleic acid encoding the GnRH-I-AP205 fusion protein is shown in SEQ ID NO: 2. This nucleic acid sequence has been codon-optimized to be more suitable for expression of the fusion protein in an *Escherichia coli* system, thereby achieving higher expression levels and biological activity. Compared with other nucleic acid sequences, it can achieve superior expression performance.

Furthermore, the present disclosure also provides an expression unit comprising the nucleic acid as described above.

The expression unit comprises the nucleic acid of the present disclosure in a single or multiple tandem form, together with a promoter and a terminator, without limitation in the present disclosure. In the present disclosure, the promoter of the expression unit is a T7 promoter, and the terminator is a T7 terminator.

Furthermore, the present disclosure provides a recombinant vector containing the nucleic acid or the expression unit as described above.

Furthermore, the recombinant expression vector refers to a nucleic acid vector, which is a recombinant DNA molecule containing the desired coding sequence and suitable nucleic acid sequences or elements that are essential for the expression of the operably linked coding gene in a specific host organism. The nucleic acid sequences or elements essential for expression in bacteria include a promoter, a ribosome-binding site, and possibly other sequences. The recombinant expression vector may be selected according to the host. In the present disclosure, the expression vector may be circular or linear, without limitation. For example, when the host is a prokaryotic organism, it may be *Escherichia coli, Bacillus, Streptomyces,* or *Cyanobacteria.* The backbone vector may be a pET series plasmid, pGEX series plasmid, pKBP series plasmid, or pcDNA series plasmid. For example, the recombinant backbone vector may be a pET series vector, and optionally, the backbone vector of the recombinant vector may be pET-21(+), pET-24(+), pET-23(+), or pET-28a(+). Preferably, in the present disclosure, the backbone vector of the recombinant vector is pET-28a(+).

The present disclosure provides a method for constructing the recombinant vector, which comprises cloning the nucleic acid fragment encoding the fusion protein, as described above, into the prokaryotic expression vector pET28a by a homologous recombination method. In some embodiments, the insertion site is located between the *Nde* I site and the *Xho* I site.

Furthermore, the present disclosure also provides a host cell that is transformed or transfected with the recombinant vector as described above, or integrated with the nucleic acid as described above into the genome of the host cell.

The type of host cells of the present disclosure includes bacteria, fungi, viruses, or animals. The bacteria include Gram-positive bacteria and Gram-negative bacteria; the Gram-negative bacteria include, but are not limited to, *Escherichia coli.* The fungi include molds, yeasts, and mushrooms; the yeasts include *Saccharomyces cerevisiae, Saccharomyces carlsbergensis, Pichia pastoris,* and *Candida.* The viruses include, but are not limited to, adenoviruses, adeno-associated viruses, lentiviruses, and prions. The animals include humans, mice, rabbits, pigs, zebrafish and so on. The expression of the nucleic acid encoding the recombinant protein in the host may be in an integrated form or in an episomal form, without limitation in the present disclosure. In the embodiments of the present disclosure, *Escherichia coli* is used as the host for the expression of fusion protein. The *Escherichia coli* strain is SHuffle^{™} T7. Compared with the previously used BL21 (DE3) strain, SHuffle^{™} T7 is more favorable for the efficient expression of fusion proteins with higher activity.

Further, the present disclosure also provides a method for preparing a GnRH-I-AP205 fusion protein, which comprises culturing the aforementioned host cell to obtain a culture product containing the GnRH-I-AP205 fusion protein.

The culture medium is LB culture medium. The culture conditions are: after inoculation, shaking at 37°C and 180 rpm until the OD₆₀₀ of the culture liquid reaching approximately 0.8, and then inducing expression of the fusion protein with IPTG. The induction conditions include expression at a temperature of 30°C overnight. The IPTG has a concentration of 1 mM. After the culture, the steps further comprise harvesting the cells and performing cell disruption. Specifically, cells are collected by centrifugation at 4,500 rpm for 15 minutes. The cell is sonicated to be disrupted for 45 minutes with cycles of 3s on and 4s off at a power of 125 W. After sonication, the lysate is centrifuged at 9,500 rpm at 4°C for 20 minutes to collect the supernatant.

The method of the present disclosure has been optimized and improved. From codon optimization to the selection of a suitable plasmid vector and host, as well as the screening of the culture medium and the optimization of culture conditions, each step cooperates and influences the others, collectively achieving further improvements in the expression level, expression efficiency, and physiological vaccine activity of the fusion protein.

Furthermore, the present disclosure also provides the culture products obtained by the method described above.

In the present disclosure, the culture products include a cultivated cell medium, centrifuged cells and a supernatant obtained by centrifugation, or further purified products obtained from the above components. The purification includes, but is not limited to, immobilized metal ion affinity chromatography. For example, the supernatant after centrifugation is purified using a nickel affinity column. Specifically the step comprises equilibrating the nickel column, then loading the centrifuged supernatant, washing with a Wash Buffer-1, and then eluting with Wash Buffers containing different concentrations of imidazole.

The Wash buffer-1 comprises 50 mM NaH₂PO₄·2H₂O, 300 mM NaCl, 10% glycerol, and 25 mM imidazole, with a pH of 8.0.

The Wash buffers containing different concentrations of imidazole are obtained by adding 50, 100, 250, or 500 mM imidazole into the Wash buffer-1 as described above.

The present disclosure further provides a vaccine comprising the culture product as described above and an adjuvant.

In the present disclosure, the vaccine is a castration vaccine, specifically a castration vaccine for male animals. Castration may be performed on male animals for example birds and mammals, such as mice, rats, cats, dogs, horses, or animals of the same family or genus as those mentioned above. Preferably, the animals are felines, canines, or rodents, for example, mice.

In the present disclosure, the adjuvants include aluminum salt adjuvant, protein-based adjuvant, nucleic acid-based adjuvant, lipid-containing adjuvant, mixed adjuvant, or adjuvant having an aggregate structure. In some embodiments of the present disclosure, the adjuvant is an aluminum hydroxide adjuvant.

The present disclosure further provides a method for preparing a vaccine, comprising mixing the purified culture product as described above with an adjuvant.

A method for animal castration, comprising administering the vaccine as described above to the aninal. The administration method may be injection.

The beneficial effects of the present disclosure are as follows:
First, AP205 is derived from a bacteriophage that infects *Acinetobacter.* The phage capsid protein has the ability to self-assemble into nanoparticles, which is non-infectious and exhibits strong antigenic immunogenicity, and represents a proprietary technology of the applicant's VLP screening platform.
Second, the antigen fused with the AP205 virus-like particle exhibits high antigenicity, capable of inducing high levels of specific antibodies in mice, rats, cats, dogs, and horses with a rapid humoral immune response.
Third, the GnRH-I-AP205_{OPT} virus-like particle of the present disclosure can be expressed extensively in *Escherichia coli* with a simple production process. After codon optimization, the yield and purity of the GnRH-I-AP205_{OPT} virus-like particles are further improved.
Fourth, the invention is suitable for industrialization. The recombinant *Escherichia coli* strain obtained in the present disclosure has the features of rapid growth, easy cultivation, simple genetic manipulation, fast reproduction, low requirements for culture conditions, low-cost culture medium, capability of high-density cultivation, and tolerance to high hydrostatic pressure, thereby facilitating industrial-scale production.

### BRIEF DESCRIPTION OF DRAWINGS

In order to more clearly illustrate the embodiments of the present disclosure or the technical solutions of the prior art, the drawings required in the description of the embodiments or the prior art are briefly introduced below. It is apparent that the drawings described below are merely exemplary. For those skilled in the art, other drawings may be derived from the provided drawings without creative efforts.

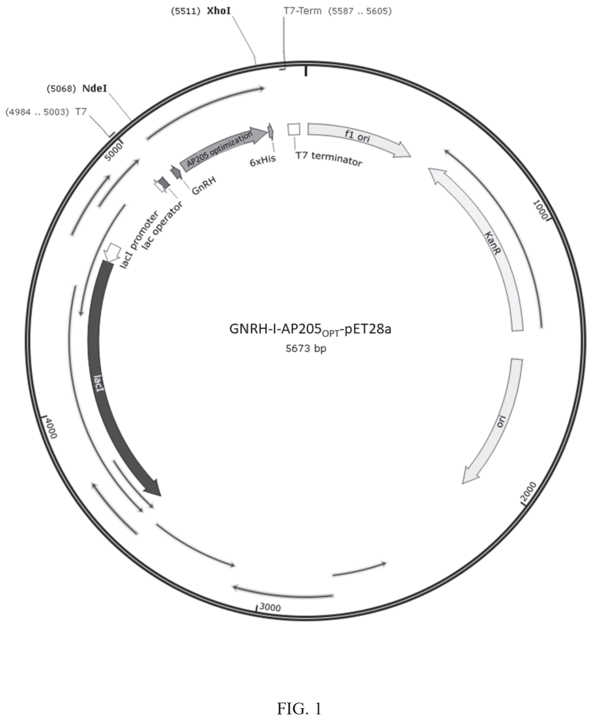

FIG. 1 shows a recombinant plasmid obtained by ligating the GnRH-I-AP205_{OPT} gene with the pET28a plasmid in the example of the present disclosure.
FIG. 2 shows a BCA quantification curve of the GnRH-I-AP205_{OPT} virus-like particles in the example of the present disclosure and the GnRH-I-AP205 virus-like particles disclosed in CN112500456B.
FIG. 3 shows an SDS-PAGE gel electrophoresis of the GnRH-I-AP205_{OPT} virus-like particles in the example of the present disclosure and the GnRH-I-AP205 virus-like particles disclosed in CN112500456B.

### DETAILED DESCRIPTION

Unless otherwise defined, all technical and scientific terms used herein have the same meanings as commonly understood by those skilled in the art to which the present disclosure pertains. The terms used in the description of the present disclosure are intended only to describe specific embodiments and are not intended to limit the present disclosure in any way. The term "and/or" as used herein refers to any and all combinations of one or more of the associated listed items.

The molecular biology experimental methods adopted in the examples of the present disclosure, such as enzyme digestion and homologous recombination, can be carried out with reference to "Molecular Cloning", 2nd Edition. The basic materials for preparing the GnRH-I-AP205_{OPT} virus-like particles of the present disclosure comprise a nucleotide sequence encoding AP205 protein, a nucleotide sequence encoding GnRH-I, a pET28a plasmid, an *Escherichia coli* SHuffle^{™} T7 strain, TIANprep Midi Plasmid Kit, LB culture medium, IPTG, kanamycin, nickel column, and imidazole and so on. The recombinant plasmid GnRH-I-A-P205_{OPT}-pET28a was synthesized by BGI Genomics.

The sequences involved in the examples:
GnRH-I-AP205_{OPT} amino acid sequence: 
MEHWSYGLRPGGGGGSMANKPMQPITSTANKIVWSDPTRLSTTFSASLLRQRVKVGIAE
LNNVSGQYVSVYKRPAPKPEGCADACVIMPNENQSIRTVISGSAENLATLKAEWETHKR
NVDTLFASGNAGLGFLDPTAAIVSSDTTA（SEQ ID NO: 2）
GnRH-I-AP205_{OPT} nucleotide sequence: 
ATGGAACACTGGAGCTACGGTTTGAGACCCGGGGGGGGCGGGGGATCCATGGCGAA
CAAACCGATGCAACCAATTACCTCTACGGCTAATAAGATCGTTTGGTCTGACCCGACC
CGTCTGTCCACGACTTTTAGCGCATCTCTGCTGCGTCAGCGCGTTAAGGTCGGTATCG
CCGAACTGAACAACGTTAGCGGCCAGTATGTGTCTGTTTATAAACGTCCGGCACCGAA
ACCGGAAGGTTGTGCTGATGCCTGCGTTATCATGCCGAACGAGAACCAGTCCATCCGT
 
ACCGTGATCTCTGGCTCTGCGGAGAACCTGGCAACCCTGAAAGCTGAATGGGAGACC
CACAAACGTAATGTTGATACCCTGTTTGCCTCTGGCAACGCAGGTCTGGGTTTTCTGG
ACCCAACCGCTGCAATTGTGTCTTCTGACACCACCGCT（SEQ ID NO: 1）
GnRH-I nucleotide sequence:
   ATGGAACACTGGAGCTACGGTTTGAGACCC (SEQ ID NO: 3)

### Example 1

A method for preparing GnRH-I-AP205_{OPT} virus-like particles is provided. The method was performed by the following steps:
(1) Construction of a recombinant plasmid: the GnRH-I-AP205_{OPT} gene was directly synthesized and inserted into a pET28a vector between the *Nde*I and *XhoI* sites by BGI Genomics to obtain a recombinant plasmid GnRH-I-AP205_{OPT}-pET28a, wherein the sequence of the AP205 gene was codon-optimized according to the sequence disclosed in Patent publication CN112500456B.
(2) Introduction of the recombinant plasmid into an expression strain: the recombinant plasmid GnRH-I-AP205_{OPT}-pET28a was introduced into an *Escherichia coli* SHuffle^{™} T7 expression strain to obtain the recombinant expression strain GnRH-I-AP205_{OPT}-pET28a-SHuffle^{™} T7.
(3) Cell culture and purification of GnRH-I-AP205_{OPT} virus-like particles: the GnRH-I-AP205_{OPT}-pET28a-SHuffle^{™} T7 recombinant expression strain was cultured and the expression was induced with IPTG to obtain GnRH-I-AP205_{OPT} virus-like particles.

Specifically, the steps of the expression and purification of GnRH-I-AP205_{OPT} virus-like particles were as follows:
(a1) Preparation of a GnRH-I-AP205_{OPT}-pET28a recombinant plasmid
   Strain stock (in glycerol) containing the plasmid was sampled with a pipette for 5 µL and inoculated into 5 mL LB culture medium (containing 50 µg/mL kanamycin), followed by incubation at 37°C for 14 hours to 16 hours with shaking. After culture, 1 mL of the cell culture liquid was taken for sequencing. The remained cell culture liquid was subjected to plasmid extraction with a plasmid mini extraction kit, and the nucleic acid concentration was measured with a nucleic acid/protein detector.
(a2) Expression of GnRH-I-AP205_{OPT} virus-like particles
   The recombinant plasmid was transformed into the SHuffle^{™} T7 expression strain. After culture with shaking, cells were concentrated by centrifugation and plated onto kanamycin-resistant plates and incubated overnight at 37°C. Colonies were scraped and inoculated into 10 mL LB culture medium containing ampicillin, and were cultured with shaking at 37°C and 180 rpm until the cell culture liquid reached an OD₆₀₀ of approximately 0.8. The cell culture liquid was then diluted 1:100 into 2,000 mL LB culture medium containing ampicillin and cultured with shaking at 37°C and 180 rpm until the cell culture liquid reached an OD₆₀₀ of approximately 0.8. Inducer IPTG was added to a final concentration of 1 mM, and the cells were cultured for expression at 30°C overnight. On the third day, cells were harvested by centrifugation at 4,500 rpm for 15 min. Cells were sonicated and broken for 45 min with cycles of 3s on and 4s off at a power of 125 W. After sonication, the lysate was centrifuged at 9,500 rpm at 4°C for 20 min and the supernatant was collected.
(a3) Purification of GnRH-I-AP205_{OPT} virus-like particles

The collected supernatant was subjected to nickel-affinity column purification. The nickel column was equilibrated with 5 column volumes of Lysis buffer (50 mM NaH₂PO₄·2H₂O, 300 mM NaCl, 10% glycerol and 10 mM imidazole, pH 8.0). After loading the sample, the column was washed with 5 column volumes of Lysis buffer to remove impurities, then washed with 5 column volumes of Wash buffer-1 (50 mM NaH₂PO₄·2H₂O, 300 mM NaCl, 10% glycerol and 25 mM imidazole, pH 8.0). Elution was then performed using Wash buffers containing imidazole at concentrations of 50, 100, 250, and 500 mM respectively with 5 column volumes, and eluates were collected.

From each collected eluate, 10 µL sample was taken and mixed with 10 µL of 2× protein electrophoresis loading buffer and heated at 100°C for 4 min to denature the proteins. Protein electrophoresis was performed, and gels were stained to observe the concentration of the target band protein in the fraction. The target protein was concentrated by ultrafiltration using a 30 kDa cut-off centrifugal filter and the buffer was exchanged into Desalting buffer (50 mM NaH₂PO₄·2H₂O and 10% glycerol, pH 7.4).

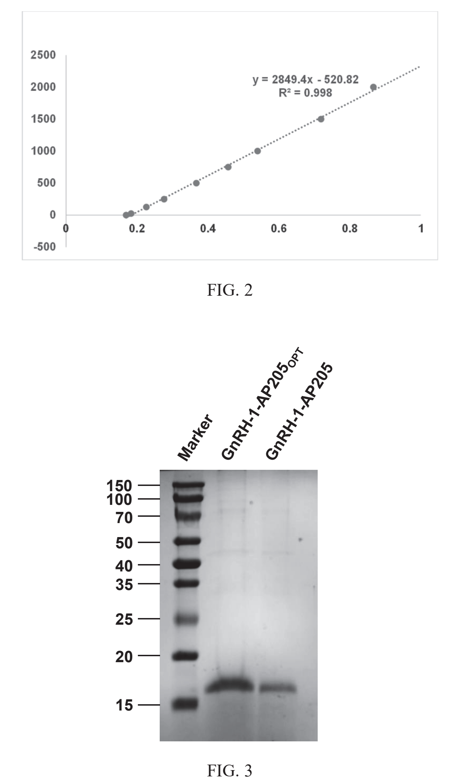

The GnRH-I-AP205 virus-like particles were prepared using the method disclosed in patent publication CN112500456B. The band homogeneity of the two virus-like particles was examined by SDS-PAGE, and the GnRH-I-AP205_{OPT} and GnRH-I-AP205 virus-like particles were quantified by BCA to compare the expression yields of the two virus-like particles (FIG. 2).

As shown in FIG. 3, the target band was detected at approximately 17 kDa for both samples; the size matched the expectation and the bands were single with no obvious contaminant bands, demonstrating that both expression and purification schemes could obtain virus-like particles of high purity. The BCA quantification raw data are shown in Table 1, and the standard curve plotted from the data in Table 1 is shown in FIG. 2. Based on the microplate reader readings, the 2× diluted GnRH-I-AP205_{OPT} sample had an absorbance of 0.7362 at 562 nm, and the 2× diluted GnRH-I-AP205 sample had an absorbance of 0.4654 at 562 nm. Substituting these values into the standard-curve equation gave concentrations of 3.153 mg/mL for GnRH-I-AP205_{OPT} and 1.611 mg/mL for GnRH-I-AP205. These results indicate that, when expressed in the same culture volume, the expression level of GnRH-I-AP205_{OPT} is substantially higher than that of GnRH-I-AP205, showing that the expression method of the present invention gives higher yield and lower cost and is therefore more suitable for preparation of GnRH-I-AP205_{OPT} virus-like particles.

**Table 1 BCA quantitative standard curve raw data**

| Concentration (µg/mL) | Absorbance (OD₅₆₂) |
|---|---|
| 2000 | 0.8662 |
| 1500 | 0.7187 |
| 1000 | 0.5399 |
| 750 | 0.457 |
| 500 | 0.3672 |
| 250 | 0.2762 |
| 125 | 0.2259 |
| 25 | 0.1832 |
| 0 | 0.1691 |

### Comparative Example 1

A method for preparing his-TEV-AP205_{OPT} recombinant protein is provided. The method was performed by the following steps:
(1) Construction of a recombinant plasmid: a His tag, a TEV protease cleavage site (amino acid sequence ENLYFQG), and a codon-optimized AP205_{OPT} gene was directly synthesized and inserted into a pET28a vector between the *BamHI* and *XhoI* restriction sites by BGI Genomics to obtain a recombinant plasmid his-TEV-AP205_{OPT}-pET28a.
(2) Introduction of the recombinant plasmid into an expression strain: the recombinant plasmid his-TEV-AP205_{OPT}-pET28a was transformed into an *Escherichia coli* SHuffle^{™} T7 expression strain to obtain the recombinant expression strain his-TEV-AP205_{OPT}-pET28a-SHuffle^{™} T7.
(3) Cell culture and purification of AP205_{OPT} virus-like particles: the his-TEV-AP205_{OPT}-pET28a-SHuffle^{™} T7 recombinant expression strain was cultured and expression was induced with IPTG to obtain his-TEV-AP205_{OPT} virus-like particles.

Specifically, the steps of the expression and purification of his-TEV-AP205_{OPT} virus-like particles were as follows:
(b1) Preparation of a his-TEV-AP205_{OPT}-pET28a recombinant plasmid
   Strain stock (in glycerol) containing the plasmid was sampled with a pipette for 5 µL and inoculated into 5 mL LB culture medium (containing 50 µg/mL kanamycin), followed by incubation at 37°C for 14 hours to 16 hours with shaking. After culture, 1 mL of the cell culture liquid was taken for sequencing. The remained cell culture liquid was subjected to plasmid extraction with a plasmid mini extraction kit, and the nucleic acid concentration was measured with a nucleic acid/protein detector.
(b2) Expression of his-TEV-AP205_{OPT} virus-like particles
   The recombinant plasmid was transformed into the SHuffle^{™} T7 expression strain. After culture with shaking, cells were concentrated by centrifugation and plated onto kanamycin-resistant plates and incubated overnight at 37°C. Colonies were scraped and inoculated into 10 mL LB culture medium containing ampicillin, and cultured at 37°C and 180 rpm with shaking until the cell culture liquid reached an OD₆₀₀ of approximately 0.8. The cell culture liquid was then diluted 1:100 into 2,000 mL LB culture medium containing ampicillin and cultured with shaking at 37°C and 180 rpm until the cell culture liquid reached an OD₆₀₀ of 0.8 to 1.0. Inducer IPTG was added to a final concentration of 1 mM, and the cells were cultured for expression overnight at 30°C. On the third day, cells were harvested by centrifugation at 4,500 rpm for 15 min. Cells were sonicated and broken for 45 min with cycles of 3s on and 4s off at a power of 125 W. After sonication, the lysate was centrifuged at 9,500 rpm for 20 min at 4°C and the supernatant was collected.
(b3) Purification of his-TEV-AP205_{OPT} virus-like particles

The collected supernatant was subjected to nickel-affinity column purification. The nickel column was equilibrated with 5 column volumes of Lysis buffer (50 mM NaH₂PO₄·2H₂O, 300 mM NaCl, 10% glycerol and 10 mM imidazole, pH 8.0). After loading the sample, the column was washed with 5 column volumes of Lysis buffer to remove impurities, then washed with 5 column volumes of Wash buffer-1 (50 mM NaH₂PO₄·2H₂O, 300 mM NaCl, 10% glycerol and 25 mM imidazole, pH 8.0). Elution were then performed using Wash buffers containing imidazole at concentrations of 50, 100, 250, and 500 mM respectively with 5 column volumes, and eluates were collected.

From each collected eluate, 10 µL sample was taken and mixed with 10 µL of 2× protein electrophoresis loading buffer and heated at 100°C for 4 min to denature the proteins. Protein electrophoresis was performed, and gels were stained to observe the concentration of the target band protein in the fraction. The target protein was concentrated by ultrafiltration using a 30 kDa cut-off centrifugal filter and the buffer was exchanged into Desalting buffer (50 mM NaH₂PO₄·2H₂O and 10% glycerol, pH 7.4).

### Comparative Example 2

A method for preparing his-MBP-TEV-GnRH-I recombinant protein is provided. The method was performed by the following steps:
(1) Construction of a recombinant plasmid: a TEV protease cleavage site and a GnRH-I gene (SEQ ID NO: 3) was directly synthesize and inserted into a pETM41 vector between the *BamH*I and *XhoI* restriction sites by BGI Genomics to obtain a recombinant plasmid his-MBP-TEV-GnRH-I-pETM41.
(2) Introduction of the recombinant plasmid into an expression strain: the recombinant plasmid his-MBP-TEV-GnRH-I-pETM41 was transformed into an *Escherichia coli* SHuffle^{™} T7 expression strain to obtain the recombinant expression strain his-MBP-TEV-GnRH-I-pETM41-SHuffle^{™} T7.
(3) Cell culture and purification of his-MBP-TEV-GnRH-I recombinant protein: the his-MBP-TEV-GnRH-I-pETM41-SHuffle^{™} T7 recombinant expression strain was cultured and expression was induced with IPTG to obtain his-MBP-TEV-GnRH-I recombinant protein.

Specifically, the steps of the expression and purification of his-MBP-TEV-GnRH-I recombinant protein were as follows:
(c1) Preparation of a his-MBP-TEV-GnRH-I-pETM41 recombinant plasmid
   Strain stock (in glycerol) containing the plasmid was sampled with a pipette for 5 µL and inoculated into 5 mL LB culture medium (containing 50 µg/mL kanamycin), followed by incubation at 37°C for 14 hours to 16 hours with shaking. After culture, 1 mL of the cell culture liquid was taken for sequencing. The remained cell culture liquid was subjected to plasmid extraction with a plasmid mini extraction kit, and the nucleic acid concentration was measured with a nucleic acid/protein detector.
(c2) Expression of his-MBP-TEV-GnRH-I recombinant protein
   The recombinant plasmid was transformed into the SHuffle^{™} T7 expression strain. After culture with shaking, cells were concentrated by centrifugation and plated onto kanamycin-resistant plates and incubated overnight at 37°C. Colonies were scraped and inoculated into 10 mL LB culture medium containing ampicillin, and were cultured with shaking at 37°C and 180 rpm until the cell culture liquid reached an OD₆₀₀ of approximately 0.8. The cell culture liquid was then diluted 1:100 into 2,000 mL LB culture medium containing ampicillin and cultured with shaking at 37°C and 180 rpm until the cell culture liquid reached an OD₆₀₀ of approximately 0.8. Inducer IPTG was added to a final concentration of 0.8 mM, and the cells were cultured for expression overnight at 30°C. On the third day, cells were harvested by centrifugation at 4,500 rpm for 15 min. Cells were sonicated and broken for 45 min with cycles of 3s on and 4s off at a power of 125 W. After sonication, the lysate was centrifuged at 9500 rpm for 20 min at 4°C and the supernatant was collected.
(c3) Purification of his-MBP-TEV-GnRH-I recombinant protein

The collected supernatant was subjected to nickel-affinity column purification. The nickel column was equilibrated with 5 column volumes of Lysis buffer (50 mM NaH₂PO₄·2H₂O, 300 mM NaCl, 10% glycerol and 10 mM imidazole, pH 8.0). After loading the sample, the column was washed with 5 column volumes of Lysis buffer to remove impurities, then washed with 5 column volumes of Wash buffer-1 (50 mM NaH₂PO₄·2H₂O, 300 mM NaCl, 10% glycerol and 25 mM imidazole, pH 8.0). Elution was then performed using Wash buffers containing imidazole at concentrations of 50, 100, 250, and 500 mM respectively, with 5 column volumes, and eluates were collected.

From each collected eluate, 10 µL sample was taken and mixed with 10 µL of 2× protein electrophoresis loading buffer and heated at 100°C for 4 min to denature the proteins. Protein electrophoresis was performed, and gels were stained to observe the concentration of the target band protein in the fraction. The target protein was concentrated by ultrafiltration using a 30 kDa cut-off centrifugal filter and the buffer was exchanged into Desalting buffer (50 mM NaH₂PO₄·2H₂O and 10% glycerol, pH 7.4).

### Comparative Example 3

A method for preparing recombinant his-TEV protease is provided. The method was performed by the following steps:
(1) Construction of a recombinant plasmid: a TEV protease gene (GenBank : MZ365307.1) was directly synthesize and inserted into a pET28a vector between the *BamH*I and *XhoI* restriction sites by BGI Genomics to obtain a recombinant plasmid his-TEV protease-pET28a.
(2) Introduction of the recombinant plasmid into an expression strain: the recombinant plasmid his-TEV protease-pET28a was transformed into an *Escherichia coli* SHuffle^{™} T7 expression strain to obtain the recombinant expression strain his-TEV protease-pET28a-SHuffle^{™} T7.
(3) Cell culture and purification of recombinant his-TEV protease: the his-TEV protease-pET28a-SHuffle^{™} T7 recombinant expression strain was cultured and expression was induced with IPTG to obtain his-TEV protease recombinant enzyme.

Specifically, the steps of the expression and purification of his-TEV protease recombinant enzyme were as follows:
(d1) Preparation of a his-TEV protease-pET28a recombinant plasmid
   Strain stock (in glycerol) containing the plasmid was sampled with a pipette for 5 µL and inoculated into 5 mL LB culture medium (containing 50 µg/mL kanamycin), followed by incubation at 37°C for 14 hours to 16 hours with shaking. After culture, 1 mL of the cell culture liquid was taken for sequencing. The remained cell culture liquid was subjected to plasmid extraction with a plasmid mini extraction kit, and the nucleic acid concentration was measured with a nucleic acid/protein detector.
(d2) Expression of recombinant his-TEV protease
   The recombinant plasmid was transformed into the SHuffle^{™} T7 expression strain. After culture with shaking, cells were concentrated by centrifugation and plated onto kanamycin-resistant plates and incubated overnight at 37°C. Colonies were scraped and inoculated into 10 mL LB culture medium containing ampicillin, and were cultured with shaking at 37°C and 180 rpm until the cell culture liquid reached an OD₆₀₀ of approximately 0.8. The cell culture liquid was then diluted 1:100 into 2000 mL LB culture medium containing ampicillin and cultured with shaking at 37°C and 180 rpm until the cell culture liquid reached an OD₆₀₀ of approximately 0.8. Inducer IPTG was added to a final concentration of 0.8 mM, and the cells were cultured for expression overnight at 30°C. On the third day, cells were harvested by centrifugation at 4500 rpm for 15 min. Cells were sonicated and broken for 45 min with cycles of 3s on and 4s off at a power of 125 W. After sonication, the lysate was centrifuged at 9500 rpm at 4°C for 20 min and the supernatant was collected.
(d3) Purification of recombinant his-TEV protease

The collected supernatant was subjected to nickel-affinity column purification. The nickel column was equilibrated with 5 column volumes of Lysis buffer (50 mM NaH₂PO₄·2H₂O, 300 mM NaCl, 10% glycerol and 10 mM imidazole, pH 8.0). After loading the sample, the column was washed with 5 column volumes of Lysis buffer to remove impurities, then washed with 5 column volumes of Wash buffer-1 (50 mM NaH₂PO₄·2H₂O, 300 mM NaCl, 10% glycerol and 25 mM imidazole, pH 8.0). Elution was then performed using Wash buffers containing imidazole at concentrations of 50, 100, 250, and 500 mM respectively with 5 column volumes, and eluates were collected.

From each collected eluate, 10 µL sample was taken and mixed with 10 µL of 2× protein electrophoresis loading buffer and heated at 100°C for 4 min to denature the proteins. Protein electrophoresis was performed, and gels were stained to observe the concentration of the target band protein in the fraction. The target protein was concentrated by ultrafiltration using a 30 kDa cut-off centrifugal filter and the buffer was exchanged into Desalting buffer (50 mM NaH₂PO₄·2H₂O and 10% glycerol, pH 7.4).

### Comparative Example 4

A method for preparing AP205_{OPT} virus-like particles and GnRH-I recombinant protein is provided. His-TEV-AP205_{OPT} virus-like particles obtained in Comparative Example 1 and his-MBP-TEV-GnRH-I recombinant protein obtained in Comparative Example 2 were each dissolved in Desalting buffer, and recombinant his-TEV protease obtained in Comparative Example 3 was added to each sample to perform cleavage. The reaction was carried out at a ratio of 10 µg TEV protease per 1 mg recombinant protein, and incubated with shaking at 30°C for 3 h. The reaction mixtures were respectively applied to nickel affinity columns. The flow-through fractions contained the GnRH-I recombinant protein or AP205_{OPT} virus-like particles, while the His-TEV protease and incompletely cleaved recombinant proteins remained bound to the nickel columns. The GnRH-I recombinant protein and the AP205_{OPT} virus-like particles were then concentrated respectively using 30 kDa cut-off centrifugal filter.

### Example 2

A method for immunization with a GnRH-I-AP205_{OPT} virus-like particle subunit vaccine was provided. As described in Example 1, the GnRH-I-AP205_{OPT} virus-like particles were mixed with an aluminum hydroxide adjuvant at a volume ratio of 1:1 for immunization. On day 0, day 14, and day 28, eight-week-old male C57BL/6 mice (five mice per group) were immunized with 50 µg of the GnRH-I-AP205_{OPT} virus-like particle vaccine. A GnRH-I-AP205 virus-like particle with aluminum hydroxide adjuvant immunization group, a GnRH-I-AP205_{OPT} without adjuvant group, an adjuvant with PBS negative control group, a AP205_{OPT} group, and a GnRH-I group were established. The antibody titers against the recombinant GnRH-I protein and the testosterone level of the mice were measured. On day 70 after immunization, the mice were sacrificed, and the teste tissues were weighed.

### Example 3

A method for determining the titer of anti-GnRH-I antibodies in mice was provided. At different time points during the experiment, serum samples were collected from immunized mice and control mice. The anti-GnRH-I IgG antibody titers were measured by ELISA as follows. The 96-well plate was coated with 100 µl per well of 2 µg/ml GnRH-I at 4°C overnight. On the next day, the plate was washed five times with PBST (1:1000), and then blocked with 300 µl of 2% BSA at 37°C for 2 hours. Then the plate was washed for five times with PBST (1:1000), and the serum sample was diluted with 2% BSA, starting from an initial dilution of 1:500 and serially two-fold diluted to 1:1000, 1:2000, 1:4000, 1:8000, 1:16000, 1:32000, 1:64000, 1:128000, and 1:256000. From the highest dilution to the lowest, 100 µl of each dilution was added per well, followed by incubation with shaking at room temperature for 45 minutes. The plate was washed five times with PBST (1:1000). HRP-labeled goat anti-mouse polyclonal antibody was used as the secondary antibody at a dilution of 1:5000, with 2% non-fat milk as the diluent. 100 µl of the secondary antibody was added per well, and incubation was performed with shaking at room temperature for 45 minutes. TMB was used for color development for 5 min to 10 min, and the reaction was terminated by adding 2 M sulfuric acid. The absorbance was read at a wavelength of 450 nm. The optical density (OD) at 450 nm was measured using an ELISA reader (BioRad Benchmark), and the maximum serum dilution corresponding to the OD₄₅₀ value was calculated based on these data.

Table 2 showed that in the group immunized with the GnRH-I-AP205_{OPT} virus-like particles without adjuvant, the average antibody titer reached 8000 on day 28 and remained at 16000 after the third booster immunization. In the group immunized with the GnRH-I-AP205_{OPT} virus-like particles with adjuvant, the average titer reached 64000 on day 28 and remained at 128000 after the third booster. In contrast, in the group immunized with the GnRH-I-AP205 virus-like particles with adjuvant, the average titer reached only 32000 on day 28 and was consistent with that of the GnRH-I-AP205_{OPT} virus-like particle with adjuvant group after the third immunization. The GnRH-I alone immunization group showed an average titer of only 2000 on day 28, while antibody titer was undetectable in the groups immunized with the AP205_{OPT} with adjuvant mixture or PBS with adjuvant mixture. The results clearly demonstrated that the GnRH-I-AP205_{OPT} virus-like particles were capable of inducing a high titer of anti-GnRH antibody, and the immunization effect was further enhanced with the administration of an adjuvant. Compared with the GnRH-I-AP205 virus-like particles disclosed in patent CN112500456B, the GnRH-I-AP205_{OPT} virus-like particles were able to stimulate a faster immune response and induce the production of corresponding antibodies more effectively, and were more suitable for the manufacture of GnRH-I-AP205_{OPT} subunit vaccine.

**Table 2**

| The average titer of 5 male mice immunized with 50 µg GnRH-I-AP205_{OPT} on day 0, day 14, and day 28 | | | | | | |
|---|---|---|---|---|---|---|
| Immunization days | GnRH-I-AP 205_{OPT} + adjuvant | GnRH-I-AP 205 + adjuvant | GnRH-I-AP 205_{OPT} + adjuvant | GnRH-I + adjuvant | AP205_{OPT} + adjuvant | PBS + adjuvant |
| day 0 | 0 | 0 | 0 | 0 | 0 | 0 |
| day 14 | 8000 | 8000 | 4000 | 2000 | 0 | 0 |
| day 28 | 64000 | 32000 | 8000 | 2000 | 0 | 0 |
| day 42 | 128000 | 128000 | 16000 | 4000 | 0 | 0 |
| day 56 | 128000 | 128000 | 16000 | 4000 | 0 | 0 |
| day 70 | 128000 | 128000 | 16000 | 2000 | 0 | 0 |

During the above experiments, serum samples were collected from the immunized mice and the control mice at various time points. The hormone level secreted by the testes in the mouse serum was determined using an ELISA kit (IBL, Hamburg, Germany).

Table 3 showed that in the mice immunized with the GnRH-I-AP205_{OPT} virus-like particles without aluminum hydroxide adjuvant, the average hormone level secreted by the testes was significantly suppressed (<2 ng/ml) on day 42 after immunization and remained below 2 ng/ml on day 70. In the mice immunized with GnRH-I-AP205_{OPT} supplemented with aluminum hydroxide adjuvant, the average testosterone level decreased to <2 ng/ml on day 28, and the average testosterone level remained stable at around 0.14 on day 42, which was approximately 39 times lower than the average hormone level in the control group mice immunized with GnRH-I + aluminum hydroxide at the same time point. Furthermore, compared with the GnRH-I-AP205 + aluminum hydroxide adjuvant immunization group, it was observed that the testosterone level of mice in the GnRH-I-AP205_{OPT} + aluminum hydroxide adjuvant immunization group were overall lower than those in the GnRH-I-AP205 + aluminum hydroxide adjuvant immunization group. The above results clearly indicated that the GnRH-I-AP205_{OPT} + aluminum hydroxide adjuvant immunization composition has a stronger inhibitory effect on testosterone level in mice.

**Table 3 Testosterone level in mouse serum**

| Immunization days | GnRH-I-AP205_{OPT} (ng/ml) | GnRH-I-AP205 + adjuvant (ng/ml) | GnRH-I-AP205_{OPT} + adjuvant (ng/ml) | GnRH-I + adjuvant (ng/ml) |
|---|---|---|---|---|
| day 0 | 5.78+0.54 | 5.72+0.47 | 5.69+0.82 | 5.62±0.73 |
| day 14 | 3.37+0.42 | 3.58+0.27 | 3.16±0.39 | 5.27±0.88 |
| day 28 | 2.25+0.36 | 1.42+0.61 | 1.24+0.72 | 5.31±0.72 |
| day 42 | 1.61±0.22 | 0.21+0.44 | 0.18±0.18 | 5.41+0.47 |
| day 56 | 1.42+0.85 | 0.14+0.55 | 0.12±0.11 | 5.47±0.11 |
| day 70 | 1.19+0.29 | 0.12±0.52 | 0.11+0.04 | 5.58±0.28 |

On day 70, the mice were sacrificed; the testes were removed and weighed, and then fixed in 4% formaldehyde.

Table 4 showed that the weight of testes from mice immunized with GnRH-I exhibited almost no reduction on day 70, whereas the weight of testes from mice immunized with the GnRH-I-AP205_{OPT} virus-like particles combined with aluminum hydroxide adjuvant decreased by 77.24%, and that of mice immunized with the GnRH-I-AP205 virus-like particles combined with aluminum hydroxide adjuvant decreased by 75.52%. These results clearly demonstrated that the vaccine comprising GnRH-I-AP205_{OPT} combined with the adjuvant has a more pronounced effect on inhibiting the growth of mouse teste.

**Table 4 Weight of testes**

| Group | before immunization | 70 days after immunization | Standard deviation of Day 70 |
|---|---|---|---|
| GnRH-I-AP205_{OPT} | 0.381 | 0.225 | 0.011 |
| GnRH-I-AP205_{OPT} + adjuvant | 0.391 | 0.089 | 0.01 |
| GnRH-I-AP205 + adjuvant | 0.384 | 0.094 | 0.012 |
| GnRH-I + adjuvant | 0.389 | 0.382 | 0.013 |
| AP205_{OPT} + adjuvant | 0.382 | 0.379 | 0.012 |

## Claims

1. A nucleic acid encoding a GnRH-I-AP205 fusion protein, comprising:
a nucleotide sequence of SEQ ID NO: 1; or
a sequence obtained by substitution, deletion, insertion, and/or replacement of one or more nucleotides of SEQ ID NO: 1; or
a sequence having more than 90% amino acid sequence identity to SEQ ID NO: 1.

2. The nucleic acid according to claim 1, wherein the nucleotide sequence of the nucleic acid is SEQ ID NO: 1.

3. An expression unit comprising the nucleic acid according to claim 3.

4. A recombinant vector comprising the nucleic acid according to claim 1 or 2, or the expression unit according to claim 3.

5. A host cell, wherein the host cell is transformed or transfected with the recombinant vector according to claim 4, or the nucleic acid according to claim 1 or 2 is integrated into the genome of the host cell.

6. The host cell according to claim 5, wherein the host cell is *Escherichia coli* SHuffle^{™} T7 strain.

7. A method for preparing a GnRH-I-AP205 fusion protein, comprising culturing the host cell according to claim 5 or 6 to obtain a culture product containing the GnRH-I-AP205 fusion protein.

8. A culture product obtained by the method according to claim 7.

9. A vaccine comprising the culture product according to claim 8 and an adjuvant.

10. A method for preparing the vaccine according to claim 9, comprising purifying the culture product according to claim 8 and mixing the culture product with an adjuvant.

11. A method for animal castration, comprising administering the vaccine according to claim 10 to the animal.
